# EUROPÄISCHE PATENTSCHRIFT

(11) **EP 2 264 413 B1**
(45) Veröffentlichungstag und Bekanntmachung des Hinweises auf die Patenterteilung: **17.12.2014**
(21) Anmeldenummer: 10005959.1
(22) Anmeldetag: 09.06.2010
(51) Int. Cl.: G01G 19/50, A61B 5/107

(54) **Personenwaage mit einer Messvorrichtung zum Ermitteln einer Körpergröße**
Weight scales with a measuring device for determining height
Balance pour personnes dotée d'un dispositif de mesure destiné à déterminer une taille corporelle

(30) Priorität: 15.06.2009 DE 102009025284
(43) Veröffentlichungstag der Anmeldung: 22.12.2010
(73) Patentinhaber: Soehnle Industrial Solutions GmbH, 71522 Backnang (DE)
(72) Erfinder: Gerster, Stephan, 53343 Wachtberg-Pech (DE)
(74) Vertreter: Hoffmann, Jürgen

(56) Entgegenhaltungen:
- CN-Y- 201 206 997
- US-A1- 2005 155 246
- US-A1- 2006 006 005

## Beschreibung

Die Erfindung betrifft eine Personenwaage mit einer Wiegeplattform, mit einer Anzeigevorrichtung und mit einer Messvorrichtung zum Ermitteln einer Körpergröße, wobei die Messvorrichtung wahlweise in eine Gebrauchsstellung oder eine kompakte Aufbewahrungsstellung überführbar ist und die Messvorrichtung in der Aufbewahrungsstellung zumindest teilweise innerhalb der Personenwaage angeordnet ist.

Waagen mit Messvorrichtungen zum Ermitteln einer Körpergröße sind in unterschiedlichen Ausführungsformen bekannt. Weit verbreitet sind Standwaagen, an denen eine etwa 2 m lange Messlatte - oft mit Rohrschellen - fest montiert ist, um zusätzlich zur Gewichtsmessung eine Messung der Körpergröße zu ermöglichen. Nachteilig an diesen Waagen mit fest installierten Messlatten ist insbesondere die schlechte Ablesbarkeit, die schlechte Transportierbarkeit und nicht zuletzt das provisorisch anmutende Design. Außerdem besteht die Gefahr, dass die ausladende Messlatte beispielsweise beim Transport, verschoben oder verbogen wird, so dass eine genaue Körpergrößenmessung dann nicht mehr möglich Ist.

US 3,808,694 offenbart eine Wiege- und Größenmessvorrichtung mit einer Messvorrichtung, die einen langen senkrechten Mast aufweist. Eine noch klobigere, automatisch arbeitende Messvorrichtung ist aus US 4,518,052 bekannt.

Aus US 2005/155246 ist eine Vorrichtung zur Messung des Gewichts und der Körpergröße einer Person bekannt. Die Vorrichtung weist eine Tafel mit zwei voneinander getrennten Displays zur Anzeige des gemessenen Gewichts einerseits und zur Anzeige der gemessen Größe andererseits auf.

Aus US 2006/006005 ist eine Vorrichtung zur Erfassung von gewichtsabhängigen Risikofaktoren bakannt, die eine Waage aufweist. Die Vorrichtung weist eine Eingabe-und-Anzeigeeinheit auf. Diese beinhaltet eine Minitastatur, einen Minidrucker, eine Uhr und einen Kalender, sowie ein LCD-Display zum Anzeigen eines Risikofaktors auf. An der Vorrichtung kann ein ausziehbares Messelineal zur Messung der Körpergröße angeordnet sein. Alternativ kann auch ein Maßband verwendet werden. Die gemessene Körpergröße muss von dem Benutzer in die Vorrichtung von Hand eingegeben werden.

Aus CN 201206997 Y ist eine Waage bekannt, in die ein flexibles und ausziehbares Maßband zum Messen einer Körpergröße integriert ist. Die Körpergröße wird unmittelbar an dem Maßband abgelesen. Eine Anzeige zum Anzeigen der Körpergröße weist die Wage nicht auf.

Es ist die Aufgabe der vorliegenden Erfindung, eine Personenwaage mit der Möglichkeit zur Krigergrößenmessung anzugeben, die einen besonders guten Bedienkomfort bietet.

Die Aufgabe wird durch eine Personenwaage gelöst, die dadurch gekennzeichnet ist, die Anzeigevorrichtung sowohl zur Anzeige eines Gewichtswertes, als auch zur Anzeige einer Körpergröße ausgebildet ist und dass die Anzeigevorrichtung beim Überführen der Messvorrichtung in die Gebrauchsstellungautomatisch in einen Modus zur Anzeige einer Körpergröße umschaltet und dass die Anzeigevorrichtung beim Überführen der Messvorrichtung in die Aufbewahrungsstellung automatisch in einen Modus zur Anzeige eines Gewichtswertes umschaltet.

Die Erfindung hat den Vorteil, dass sich die Personenwaage automatisch an den aktuellen Benutzungsmodus anpasst. Inshesondere kann so vermieden werden, dass eine Gewichtsmessung durch eine gleichzeitige Körpergrößenmessung verfälscht wird. Die Anzeigevorrichtung kann jedoch zusätzlich manuell wahlweise zwischen einem Modus zur Anzeige eines Gewichtswertes und einem Modus zur Anzeige einer Körpergröße umschaltbar sein.

Die Erfindung hat den Vorteil, dass die Messvorrichtung gegen Verschmutzung und Beschädigung gut geschützt untergebracht ist, wenn sie nicht gebraucht oder transportiert wird. Darüber hinaus braucht die erfindungsgemäße Waage nicht größer als Waagen ohne Körpergrößenmesseinrichtung zu sein. Insbesondere kann die Messvorrichtung zur Bestimmung der Körpergröße in der Aufbewahrungsstellung in einem ohnehin vorhandenen Gehäuse oder Gehäuseteil untergebracht sein. Dieses braucht, je nach Ausprägung der Messvorrichtung gar nicht oder nur unwesentlich größer zu sein, als übliche Gehäuse von Waagen ohne Körpergrößenmesseinrichtung.

Bei einer besonderen Ausführung ist vorgesehen, dass die Messvorrichtung in der Aufbewahrungsstellung innerhalb des Gehäuses der Personenwaage angeordnet ist, in dem sich auch andere für die Wiegefunktion erforderliche Bauteile, wie Wägezellen oder Gestänge oder Versorgungsleitungen oder Zuleitungen, beispielsweise solche zur Anzeigevorrichtung, befinden.

Bei einer besonderen Ausführungsform, bei der der Benutzer während des Wiegvorganges nicht extrem nach unten Schauen muss, um die Anzeige abzulesen, ist vorgesehen, dass die Anzeigevorrichtung von einer Tragevorrichtung in einer zum relativ zur Wiegeplattform erhöhten Position gehalten ist und dass die Messvorrichtung zum Ermitteln einer Körpergröße in der Aufbewahrungsstellung zumindest teilweise in der Tragevorrichtung angeordnet ist und/oder anordenbar ist. Diese Waage hat den Vorteil, dass ein ausbalanciertes und ruhiges Stehen gewährleistet ist, was der Genauigkeit der Gewichtsmessung zu Gute kommt. Außerdem ist die Anzeige leichter ablesbar. Bei einer vorteilhaften Ausführung ist die Anzeigevorrichtung hüfthoch, insbesondere ca. 90 bis 1,30 m hoch, positioniert.

Die Tragevorrichtung kann beispielsweise als Rohr und/oder als Säule und/der als Geländer ausgebildet sein. Bei einer besonderen Ausführung ist eine Abdeckhaube oder ein Deckel vorgesehen, um die Messvorrichtung in der Aufbewahrungsstellung vollständig zu schützen.

Eine komfortabel zu handhabende und dennoch weitgehend einfach transportierbare Ausführung der erfindungsgemäßen Personenwaage weist eine hüfthohe, insbesondere ca. 90 bis 1,30 m hohe, Tragevorrichtung auf. In dieser Ausführung lässt sich eine Messvorrichtung zum Ermitteln einer Körpergröße derart unterbringen, dass sie schnell und einfach, ohne Bücken oder andere aufwändige Tätigkeiten, in die Gebrauchstellung überführt werden kann. Insbesondere kann vorgesehen sein, dass sich die Messvorrichtung nach oben, beispielsweise nach entfernen oder wegklappen eines Deckels, aus der Tragevorrichtung zumindest teilweise herausziehen oder herausklappen lässt.

Vorteilhafter Weise kann, insbesondere um die Messvorrichtung in der Aufbewahrungsstellung vor Beschädigungen zu schützen vorgesehen sein, dass die Messvorrichtung in der Aufbewahrungsstellung vollständig innerhalb der Tragevorrichtung angeordnet ist.

Bei einer Ausführungsform, die ein schnelles Wechseln zwischen Aufbewahrungsstellung und Gebrauchstellung erlaubt, ist vorgesehen, dass die Messvorrichtung zur Überführung in die Gebrauchstellung aus der Tragevorrichtung - zumindest teilweise - ausziehbar oder ausklappbar ist. Beispielsweise kann die Messvorrichtung teleskopisch ausziehbar sein,

Bei einer besonderen Ausführung der erfindungsgemäßen Waage ist die Anzeigevorrichtung sowohl zur Anzeige eines Gewichtswertes, als auch zur Anzeige einer Körpergröße ausgebildet. Dies hat den Vorteil, dass insgesamt lediglich eine einzige Anzeige benötigt wird.

Bei einer besonders präzise Messungen erlaubenden Ausführungsform ist die Messvorrichtung als Messschieber und/oder als digitaler Messschieber ausgebildet. Insbesondere kann vorgesehen sein, dass die Messvorrichtung einen ausziehbaren Messschenkel aufweist, an dem beispielsweise ein verschiebbarer und/oder klappbarer Anschlag angeordnet sein kann.

Generell kann die Messvorrichtung nach einer Vielzahl von Längenmessverfahren arbeiten. Beispielsweise kann vorgesehen sein, dass die Messvorrichtung nach einem optischen Längenmessverfahren und/oder nach einem Ultraschall-Messverfahren arbeitet.

Bei einer besonders kompakt ausbildbaren Ausführungsform ist vorgesehen, dass die Tragevorrichtung zusätzlich auch als Teil der Messvorrichtung fungiert. Beispielsweise kann die Tragsäule, die auch die Anzeigevorrichtung trägt, gleichzeitig als einer von zwei Messschenkeln eines - vorzugsweise digitalen - Messschiebers sein. Insbesondere kann hierbei die Messelektronlk platzsparend in der Tragevorrichtung angeordnet sein.

In der Zeichnung ist der Erfindungsgegenstand schematisch dargestellt und wird anhand der Figuren nachfolgend beschrieben, wobei gleich wirkende Elemente mit denselben Bezugszeichen versehen sind. Dabei zeigen:
- Fig. 1: eine erfindungsgemäße Personenwaage mit einer Messvorrichtung zum Ermitteln einer Körpergröße in der Aufbewahrungsstellung,
- Fig. 2: die erfindungsgemäße Personenwaage mit der Messvorrichtung Gebrauchsstellung, und
- Fig. 3: eine weitere erfindungsgemäße Personenwaage mit der Messvorrichtung in einer Gebrauchsstellung.

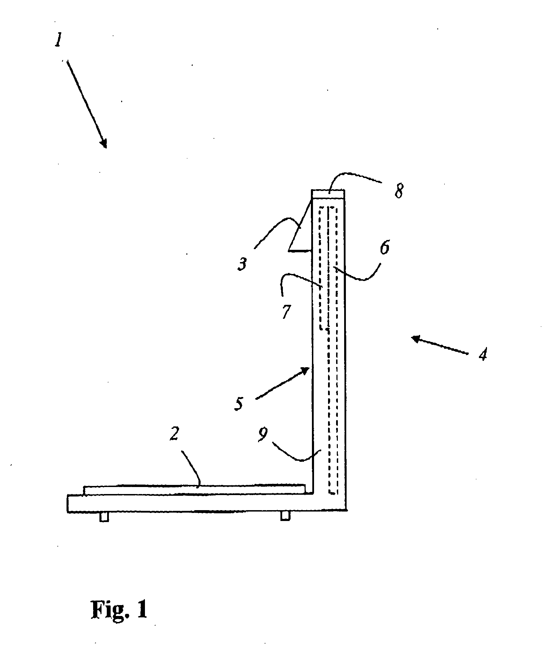

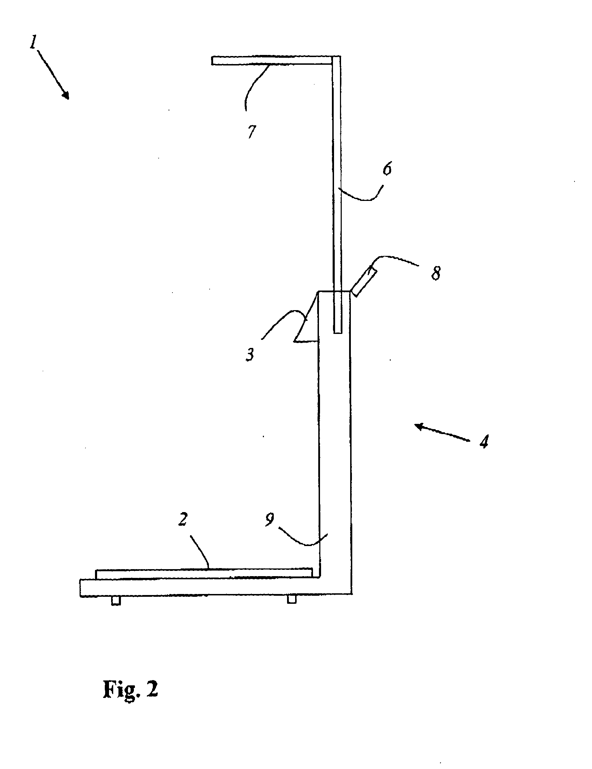

Fig. 1 zeigt eine erfindungsgemäße Personenwaage 1 mit einer Wiegeplattform 2 und mit einer Anzeigevorrichtung 3. Die Anzeigevorrichtung 3 ist an einer Tragevorrichtung 4, die als Säule 9 ausgebildet ist, in einer relativ zur Wiegeplattform 2 erhöhten Position, nämlich hüfthoch, befestigt. Die Personenwaage 1 weist eine Messvorrichtung 5 zum Ermitteln einer Körpergröße auf, die sich in der gezeigten Darstellung in der Aufbewahrungsposition und teilweise in der Tragevorrichtung 4 befindet. Die Messvorrichtung 5 zum Ermitteln einer Körpergröße ist als digitaler Messschieber ausgeführt und weist einen Messschenkel 6 und einen klappbar an dem Messschenkel 6 befestigten Anschlag 7 auf. Die Tragvorrichtung 4 selbst dient als zweiter Messschenkel der Messvorrichtung 5. Die Tragevorrichtung 4 weist einen Deckel 8 auf, der zum Überführen der Messvorrichtung 5 zum Ermitteln einer Körpergröße von der Aufbewahrungsstellung in die Gebrauchsstellung verschwenkt werden kann, was ein Herausziehen des Messschenkels 6 samt dem Anschlag 7 erlaubt. Dies ist in Fig. 2 dargestellt. In der Gebrauchstellung wird der zunächst bis in eine Maximalposition ausgezogene Messschenkel soweit nach unten bewegt, bis der Anschlag 7 auf dem Kopf der zu vermessenden, auf der Wiegeplattform aufrecht stehenden Person aufliegt. Die Anzeigevorrichtung 3, die bereits ausgelöst durch das Öffnen des Deckels 8 in den Modus zum Anzeigen einer Körpergröße wechselte, zeigt die zur aktuellen Stellung des Anschlags 7 zugehörige Körpergröße an. Beim Schließen des Deckels 8 schaltet die Anzeigevorrichtung 3 automatisch in den Modus zur Anzeige eines Gewichtswertes.

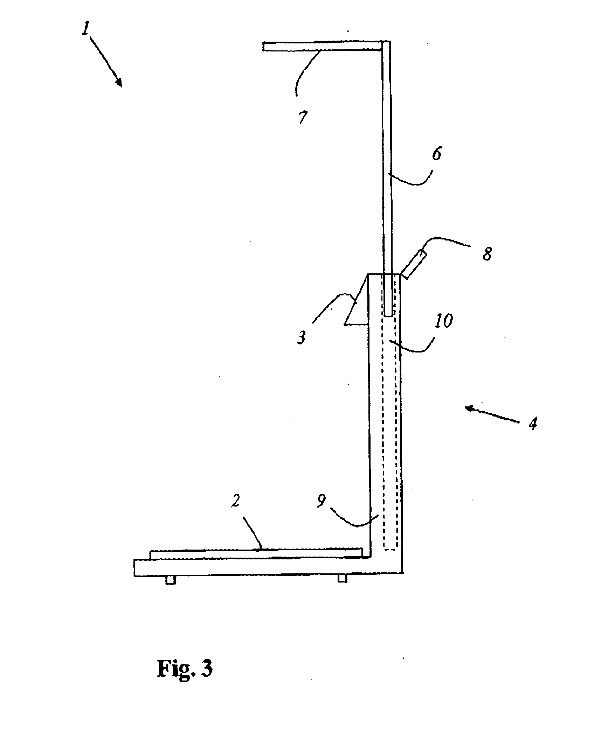

Fig. 3 zeigt eine andere erfindungsgemäße Personenwaage 1, bei der im Gegensatz zur in den Figuren 1 und 2 dargestellten Personenwaage 1 die Tragevorrichtung 4 nicht als Teil der Messvorrichtung 5 ausgebildet ist, sondern bei der die Messvorrichtung 5 als eigenständig funktionierender, digitaler Messschieber mit einem eigenständigen zweiten Messschenkel 10 ausgebildet ist. Diese Ausführung hat den Vorteil, dass die Messvorrichtung 5 einfach ausgewechselt oder nachträglich eingebaut werden kann. Bei dieser Ausführung dient die Tragevorrichtung ausschließlich als Gehäuse u.a. für Zuleitungen und die Messvorrichtung 5 und zum Halten der Anzeigevorrichtung 3.

### Bezugszeichenliste

- 1: Personenwaage
- 2: Wiegeplattform
- 3: Anzeigevorrichtung
- 4: Tragevorrichtung
- 5: Messvorrichtung zum Ermitteln einer Körpergröße
- 6: Messschenkel
- 7: Anschlag
- 8: Deckel
- 9: Säule
- 10: zweiter Messschenkel

## Patentansprüche

1. Personenwaage mit einer Wiegeplattform, mit einer Anzeigevorrichtung und mit einer Messvorrichtung zum Ermitteln einer Körpergröße, wobei die Messvorrichtung wahlweise in eine Gebrauchsstellung oder eine kompakte Aufbewahrungsstellung überführbar ist und die Messvorrichtung in der Aufbewahrungsstellung zumindest teilweise innerhalb der Personenwaage angeordnet ist, **dadurch gekennzeichnet, dass** die Anzeigevorrichtung sowohl zur Anzeige eines Gewichtswertes, als auch zur Anzeige einer Körpergröße ausgebildet ist und dass die Anzeigevorrichtung beim Überführen der Messvorrichtung in die Gebrauchsstellung automatisch in einen Modus zur Anzeige einer Körpergröße umschaltet und dass die Anzeigevorrichtung beim Überführen der Messvorrichtung in die Aufbewahrungsstellung automatisch in einen Modus zur Anzeige eines Gewichtswertes umschaltet.

2. Personenwaage nach Anspruch 1, **dadurch gekennzeichnet, dass** die Anzeigevorrichtung von einer Tragevorrichtung in einer relativ zur Wiegeplattform erhöhten Position gehalten ist und dass die Messvorrichtung zum Ermitteln einer Körpergröße in der Aufbewahrungsstellung zumindest teilweise in der Tragevorrichtung angeordnet ist und/oder anordenbar ist.

3. Personenwaage nach Anspruch 2, **dadurch gekennzeichnet, dass** die Messvorrichtung in der Aufbewahrungsstellung vollständig innerhalb der Tragevorrichtung angeordnet ist.

4. Personenwaage nach einem der Ansprüche 2 bis 3, **dadurch gekennzeichnet, dass** die Messvorrichtung zur Überführung in die Gebrauchstellung aus der Tragevorrichtung - zumindest teilweise - ausziehbar ist.

5. Personenwaage nach einem der Ansprüche 1 bis 4, **dadurch gekennzeichnet, dass** die Messvorrichtung teleskopisch ausziehbar und/oder ausklappbar ist.

6. Personenwaage nach einem der Ansprüche 1 bis 5, **dadurch gekennzeichnet, dass** die Anzeigevorrichtung manuell wahlweise zwischen dem Modus zur Anzeige eines Gewichtswertes und dem Modus zur Anzeige einer Körpergröße umschaltbar ist.

7. Personenwaage nach einem der Ansprüche 1 bis 6, **dadurch gekennzeichnet, dass** die Messvorrichtung als Messschieber und/oder als digitaler Messschieber ausgebildet ist.

8. Personenwaage nach einem der Ansprüche 1 bis 7, **dadurch gekennzeichnet, dass** die Messvorrichtung einen ausziehbaren und/oder ausklappbaren Messschenkel aufweist und/oder an dem Messschenkel ein verschiebbarer und/oder klappbarer Anschlag angeordnet ist.

9. Personenwaage nach einem der Ansprüche 1 bis 8, **dadurch gekennzeichnet, dass** die Messvorrichtung nach einem optischen Längenmessverfahren arbeitet und/oder dass die Messvorrichtung nach einem Ultraschall-Messverfahren arbeitet.

10. Personenwaage nach einem der Ansprüche 2 bis 9, **dadurch gekennzeichnet, dass** die Tragevorrichtung zusätzlich auch als Teil der Messvorrichtung fungiert.

11. Personenwaage nach einem der Ansprüche 2 bis 10, **dadurch gekennzeichnet, dass** die Tragevorrichtung als Rohr und/oder als Säule und/der als Geländer ausgebildet ist.

12. Personenwaage nach einem der Ansprüche 2 bis 11, **dadurch gekennzeichnet, dass** die Tragevorrichtung hüfthoch, insbesondere ca. 90 bis 1,30 m hoch, ausgebildet ist und/oder dass die Anzeigevorrichtung hüfthoch, insbesondere ca. 90 bis 1,30 m hoch, positioniert ist.

13. Personenwaage nach einem der Ansprüche 2 bis 12, **dadurch gekennzeichnet, dass** die Tragevorrichtung (4) einen Deckel (8) aufweist, der zum Überführen der Messvorrichtung (5) zum Ermitteln einer Körpergröße von der Aufbewahrungsstellung in die Gebrauchsstellung verschwenkt werden kann und beim Schließen des Deckels (8) die Anzeigevorrichtung (3) automatisch in einen Modus zur Anzeige eines Gewichtswerts schaltet.

## Claims

1. Set of weighing scales with a weighing platform, having a display apparatus and having a measuring apparatus for ascertaining a body height, wherein the measuring apparatus is optionally transferrable into a use position or a compact storage position and the measuring apparatus in the storage position is arranged at least partially within the set of weighing scales, **characterized in that** the display apparatus is configured both for displaying a weight value and for displaying a body height, and **in that** the display apparatus, when transferring the measuring apparatus into the use position, automatically switches into a mode for displaying a body height, and **in that** the display apparatus, when transferring the measuring apparatus into the storage position, automatically switches into a mode for displaying a weight value.

2. Set of weighing scales according to Claim 1, **characterized in that** the display apparatus is held in an elevated position relative to the weighing platform by a carrying apparatus and **in that** the measuring apparatus for ascertaining a body height in the storage position is arranged and/or arrangeable at least partially within the carrying apparatus.

3. Set of weighing scales according to Claim 2, **characterized in that** the measuring apparatus in the storage position is arranged completely within the carrying apparatus.

4. Set of weighing scales according to one of Claims 2 or 3, **characterized in that** the measuring apparatus is - at least partially - extendable out of the carrying apparatus for being transferred into the use position.

5. Set of weighing scales according to one of Claims 1 to 4, **characterized in that** the measuring apparatus is telescopically extendable and/or foldable.

6. Set of weighing scales according to one of Claims 1 to 5, **characterized in that** the display apparatus is manually switchable optionally between the mode for displaying a weight value and the mode for displaying a body height.

7. Set of weighing scales according to one of Claims 1 to 6, **characterized in that** the measuring apparatus is configured as a vernier caliper and/or as a digital caliper.

8. Set of weighing scales according to one of Claims 1 to 7, **characterized in that** the measuring apparatus has an extendable and/or foldable measuring leg and/or a displaceable and/or foldable stop is arranged on the measuring leg.

9. Set of weighing scales according to one of Claims 1 to 8, **characterized in that** the measuring apparatus operates according to an optical length measuring method and/or **in that** the measuring apparatus operates according to an ultrasound measuring method.

10. Set of weighing scales according to one of Claims 2 to 9, **characterized in that** the carrying apparatus additionally also functions as part of the measuring apparatus.

11. Set of weighing scales according to one of Claims 2 to 10, **characterized in that** the carrying apparatus is configured as a tube and/or as a column and/or as a handrail.

12. Set of weighing scales according to one of Claims 2 to 11, **characterized in that** the carrying apparatus is configured to be hip-high, in particular to have a height of approximately 90 to 1.30 m, and/or **in that** the display apparatus is positioned such that it is hip-high, in particular at a height of approximately 90 to 1.30 m.

13. Set of weighing scales according to one of Claims 2 to 12, **characterized in that** the carrying apparatus (4) has a lid (8), which can be pivoted for transferring the measuring apparatus (5) for ascertaining a body height from the storage position into the use position and the display apparatus (3) switches automatically into a mode for displaying a weight value when the lid (8) is being closed.

## Revendications

1. Pèse-personne doté d'un plateau de balance, d'un dispositif d'affichage et d'un dispositif de mesure permettant de calculer une taille corporelle, le dispositif de mesure pouvant être amené au choix dans une position d'utilisation ou dans une position de rangement compacte et le dispositif de mesure étant disposé dans la position de rangement au moins en partie à l'intérieur du pèse-personne, **caractérisé en ce que** le dispositif d'affichage sert tant à afficher une valeur de poids qu'à afficher une taille corporelle et que le dispositif d'affichage bascule automatiquement, lors du passage du dispositif de mesure dans la position d'utilisation, dans un mode d'affichage d'une taille corporelle et que le dispositif d'affichage bascule automatiquement, lors du passage du dispositif de mesure dans la position de rangement, dans un mode d'affichage d'une valeur de poids.

2. Pèse-personne selon la revendication 1, **caractérisé en ce que** le dispositif d'affichage est maintenu dans une position surélevée par rapport au plateau de balance par un dispositif de support et que le dispositif de mesure servant à calculer une taille corporelle est disposé et/ou peut être agencé dans la position de rangement au moins en partie dans le dispositif de support.

3. Pèse-personne selon la revendication 2, **caractérisé en ce que** le dispositif de mesure est entièrement disposé à l'intérieur du dispositif de support dans la position de rangement.

4. Pèse-personne selon l'une quelconque des revendications 2 à 3, **caractérisé en ce que** le dispositif de mesure peut être retiré - au moins en partie - du dispositif de support pour être amené dans la position d'utilisation.

5. Pèse-personne selon l'une quelconque des revendications 1 à 4, **caractérisé en ce que** le dispositif de mesure peut être tiré et/ou rabattu télescopiquement.

6. Pèse-personne selon l'une quelconque des revendications 1 à 5, **caractérisé en ce que** le dispositif d'affichage peut être basculé manuellement au choix entre le mode d'affichage d'une valeur de poids et le mode d'affichage d'une taille corporelle.

7. Pèse-personne selon l'une quelconque des revendications 1 à 6, **caractérisé en ce que** le dispositif de mesure prend la forme d'un pied à coulisse et/ou d'un pied à coulisse numérique.

8. Pèse-personne selon l'une quelconque des revendications 1 à 7, **caractérisé en ce que** le dispositif de mesure comporte une patte de mesure rétractable et/ou rabattable et/ou une butée pouvant être coulissée et/ou rabattue étant disposée au niveau de la patte de mesure.

9. Pèse-personne selon l'une quelconque des revendications 1 à 8, **caractérisé en ce que** le dispositif de mesure fonctionne selon un procédé de mesure de longueur optique et/ou que le dispositif de mesure fonctionne selon un procédé de mesure par ultrasons.

10. Pèse-personne selon l'une quelconque des revendications 2 à 9, **caractérisé en ce que** le dispositif de support sert en outre de partie du dispositif de mesure.

11. Pèse-personne selon l'une quelconque des revendications 2 à 10, **caractérisé en ce que** le dispositif de support prend la forme d'un tube et/ou d'une colonne et/ou d'une rambarde.

12. Pèse-personne selon l'une quelconque des revendications 2 à 11, **caractérisé en ce que** le dispositif de support est positionné à hauteur de hanche, notamment à une hauteur d'environ 90 à 1,30 m et/ou que le dispositif d'affichage est réalisé à hauteur de hanche, notamment à une hauteur d'environ 90 à 1,30 m.

13. Pèse-personne selon l'une quelconque des revendications 2 à 12, **caractérisé en ce que** le dispositif de support (4) comporte un cache (8) servant à basculer le dispositif de mesure (5) servant à calculer une taille corporelle de la position de rangement dans la position d'utilisation et pouvant être pivoté et le dispositif d'affichage (3) étant automatiquement basculé dans un mode d'affichage d'une valeur de poids lors de la fermeture du cache (8).
